# EUROPÄISCHE PATENTSCHRIFT

(11) **EP 0 614 969 B1**
(45) Veröffentlichungstag und Bekanntmachung des Hinweises auf die Patenterteilung: **28.11.2001**
(21) Anmeldenummer: 94102613.0
(22) Anmeldetag: 22.02.1994
(51) Int. Cl.: C12N 7/00, C12N 5/10

(54) **Verwendung von mit Herpesvirus saimiri transformierten Lymphozyten in einem Verfahren zur Vermehrung von Immunschwäche verursachenden Viren vom HIV-Typ**
Use of Herpes saimiri transformed lymphocytes in a process of multiplication of immune deficiency inducing virus of the HIV type
Utilisation de lymphocytes transformés par le virus Herpes saimiri dans un procédé de multiplication de virus occasionnant une immunodéficience du type VIH

(30) Priorität: 12.03.1993 DE 4307970
(43) Veröffentlichungstag der Anmeldung: 14.09.1994
(73) Patentinhaber: HOECHST AKTIENGESELLSCHAFT, 65929 Frankfurt am Main (DE)
(72) Erfinder: Nick, Sigrid, Dr., D-91301 Forchheim (DE); Fickenscher, Helmut, Dr., D-90765 Fürth-Mannhof (DE); Biesinger-Zwosta, Brigitte, Dr., D-91080 Uttenreuth (DE); Jahn, Gerhard, Prof. Dr., D-72076 Tübingen (DE); Fleckenstein, Bernhard, Prof. Dr., D-91369 Wiesenthau (DE)
(74) Vertreter: Jacobi, Markus Alexander, Dr.

(56) Entgegenhaltungen:
- WO-A-90/04020
- THE JOURNAL OF EXPERIMENTAL MEDICINE, Bd. 176, Nr. 3, 1.September 1992 Seiten 909-913, MITTRÜCKER H-W. ET AL. 'CD2-mediated Autocrine Growth of Herpes Virus Saimiri-transformed Humen T Lymphocytes'
- CHEMICAL ABSTRACTS, vol. 122, no. 17, 24.April 1995 Columbus, Ohio, US; abstract no. 210784, FICKENSCHER, HELMUT ET AL 'Viral genes involved in stable growth transformation of human T-lymphocytes' & VIRUS STRATEGIES (1993), 401-6. EDITOR(S): DOERFLER, WALTER;BOEHM, PETRA. PUBLISHER: VCH, WEINHEIM, GERMANY. CODEN: 60ZKAT, 1993
- VIRUS RESEARCH, Bd. 19, 1991 AMSTERDAM, Seiten 153-162, EBERLEIN B. ET AL. 'Expression of human immunodeficiency virus (HIV) in naturally infected peripheral blood mononuclear cells ...'
- PROCEEDINGS OF THE NATIONAL ACADEMY OF SCIENCES OF USA, Bd. 89, Nr. 7, 1.April 1992 WASHINGTON US, Seiten 3116-3119, BIESINGER B. ET AL. 'Stable growth transformation of human T lymphocytes by Herpesvirus saimiri'
- VIROLOGY 194 (2). 1993. 875-877. CODEN: VIRLAX ISSN: 0042-6822, NICK S ET AL 'HERPESVIRUS SAIMIRI TRANSFORMED HUMAN T CELL LINES A PERMISSIVE SYSTEM FOR HUMAN IMMUNODEFICIENCY VIRUSES.'
- J IMMUNOL 151 (3). 1993. 1184-1192. CODEN: JOIMA3 ISSN: 0022-1767, BROKER B M ET AL 'IMMORTALIZATION OF HUMAN T CELL CLONES BY HERPESVIRUS-SAIMIRI SIGNAL TRANSDUCTION ANALYSIS REVEALS FUNCTIONAL CD3 CD4 AND IL-2 RECEPTORS.'
- 24TH MEETING OF THE SOCIETY FOR IMMUNOLOGY, LEIPZIG, GERMANY, SEPTEMBER 30-OCTOBER 2, 1993. IMMUNOBIOLOGY 189 (1-2). 1993. 14. ISSN: 0171-2985, FICKENSCHER H ET AL 'Viral expression during growth transformation of human T-lymphocytes by herpesvirus saimiri.'

## Beschreibung

Es hat sich in den letzten Jahren herausgestellt, daß die Infektion von Immunschwäche verursachenden Viren vom HIV-Typ ein gravierendes Problem darstellt, an dessen Lösung intensiv geforscht wird.

Im Rahmen der weltweiten Forschungsanstrengungen wurde herausgefunden, daß die Immunschwächeerkrankung, die als.AIDS bezeichnet wird, nicht nur von einem einzigen, zu den RNA-Viren gehörenden Virustyp verursacht wird, sondern daß die Erkrankung von wenigstens zwei größeren Gruppen von HIV-Viren verursacht wird. Die einzelnen Erreger weisen eine hohe genetische Variabilität auf und können meist in einer der bereits bekannten Gruppen von HIV-Viren zugeordnet werden.

Da die HIV-Viren nicht über einen eigenen Stoffwechsel verfügen, erfolgt die Vermehrung im Labor zu Forschungs- oder Diagnosezwecken mit Hilfe von Zellinien.

Zur Vermehrung von Immunschwäche verursachenden Viren vom HIV-Typ sind verschiedene Zellinien bekannt. Es hat sich jedoch herausgestellt, daß verschiedene Virusstämme bzw. Virusisolate, insbesondere solche, die von asymptomatischen seropositiven Patienten stammen, nicht oder nur sehr schlecht in den bekannten Zellinien vermehrt werden können. Derartige Viren werden deshalb häufig auf primären Lymphozytenkulturen gezogen, die jeweils frisch hergestellt werden müssen. Die dabei erzielte Virusausbeute ist gering und die Vermehrungseigenschaften der primären Zellen sind unterschiedlich.

Aufgabe der vorliegenden Erfindung ist es daher, ein Verfahren zur Vermehrung von Immunschwäche verursachenden Viren vom HIV-Typ bereitzustellen, mit dem insbesondere sich nur schwach vermehrende HIV-Stämme oder primäre klinische Isolate vermehrt werden können.

Das erfindungsgemäße Verfahren ist dadurch gekennzeichnet, daß die Viren mit Hilfe von mit Herpesvirus saimiri transformierten Lymphozyten vermehrt werden.

Das Herpesvirus saimiri induziert T-Zellymphome in verschiedenen Arten von Neuwelt-Affen und in Kaninchen. In südamerikanischen Totenkopfäffchen (Saimiri ciureus) ist das Herpesvirus saimiri häufig aufzufinden, aber nicht pathogen. Stämme von Herpesvirus saimiri, Untergruppe C, transformieren wirksam menschliche Lymphozyten aus dem Thymus oder dem peripheren Blut von menschlichen Spendern zu sich ständig vermehrenden T-Zellinien (Proc. Natl. Acad. Sci. USA, Vol. 89, S. 3116-3119, April 1992).

Bei den im Rahmen des erfindungsgemäßen Verfahrens verwendbaren Lymphozyten handelt es sich bevorzugt um Lymphozyten, die aus dem menschlichen Thymus, dem menschlichen peripheren Blut oder menschlichem Nabelschnurblut herstammen.

Geeignete Lymphozyten könnten beispielsweise dadurch hergestellt werden, daß die Zellen mit Hilfe von Zentrifugation durch Histopaque Dichtegradienten (1,1 g/ml; Pharmacia) isoliert werden, um einkernige Zellen zu erhalten, die anschließend mit Phytohemagglutinin P stimuliert werden. Andererseits können bei der Gewinnung aus Blut die Erythrozyten durch Dextransedimentation abgetrennt und die Leukozyten aus dem Überstand sedimentiert werden. Thymozyten werden aus zerkleinertem Thymusgewebe gewonnen, das üblicherweise von Kindern, die einer Herzoperation unterzogen wurden, erhalten wird.

Stimulierte und nicht stimulierte Zellen wurden in geeigneten Kulturgefäßen bei einer Dichte von 1x10⁶ Zellen pro ml angezogen und mit 10⁴ bis 10⁶ infektiösen Dosen des Herpesvirus saimiri inokuliert. Die Phytohemagglutininstimulierten Zellen wurden zusätzlich mit rekombinantem Interleukin 2 (50 U/ml) supplementiert. Die Bildung von infektiösem Virus wurde überprüft anhand von Kokultivierung der Lymphozyten mit Affennierenzellen.

Zellen weisen an den Oberflächen Strukturen auf, die bestimmte Funktionen haben, wie z.B. verschiedene Rezeptoren. Diese Oberflächenstrukturen werden zur Identifizierung der Zellen verwendet.

Es wurde nun herausgefunden, daß für die Vermehrung von Viren vom HIV-Typ solche Zellinien besonders geeignet sind, die an der Oberfläche das sogenannte CD4⁺-Molekül aufweisen.

Im Rahmen des erfindungsgemäßen Verfahrens werden bevorzugt mit Herpesvirus saimiri transformierte Zellen eingesetzt, die ausgewählt sind aus der Gruppe von folgenden Zellinien: PB-W, CB-15 und Lucas (PNAS, **89**, S. 3116-3119, April 1992) oder PB-M, CB-23 und Kesting (Simmer, B., Berthold, S., Biesinger, B, Müller-Fleckenstein, I., Kalden, J., Platzer, E., Fleckenstein, B., Manuskript in Vorbereitung).

Besonders vorteilhaft kann das erfindungsgemäße Verfahren zur Vermehrung von solchen Viren vom HIV-Typ verwendet werden, die sich sonst nur sehr schwer vermehren lassen. Manche Stämme bereiten erhebliche Schwierigkeiten bei der Anzüchtung im Labor. Dies gilt insbesondere für solche Stämme, die aus Patienten stammen, die zwar klinisch ohne Symptome sind, jedoch seropositiv sind.

Das vorliegende Verfahren stellt ein sehr vorteilhaftes Kultivierungssystem für Immunschwäche verursachende Viren dar. Mit Herpesvirus saimiri transformierte menschliche lymphoide Zellen, die CD4-positiv sind, sind gegenüber einer Infektion mit den Prototypen HIV-Stämmen HIV-1_{IIIB} und HIV-2_{ROD} hochempfindlich und produzieren dabei große Virus mengen. Diese Zellinien sind ganz besonders für die Vermehrung von HIV-2-Isolaten geeignet, die ein begrenztes Zellspektrum haben und von asymptomatischen Patienten stammen.

Darüber hinaus bieten die mit Herpesvirus saimiri transformierten Zellen Vorteile, wenn sie mit Spenderlymphozytenzellen aus peripherem Blut kokultiviert werden. Bei der Kokultivierung werden aus einem, bevorzugt HIV-positiven, Spender gewonnene Lymphozyten zusammen mit anderen Zellen bzw. Zellinien angezogen. Die mit Herpesvirus saimiri transformierten Zellen zeigen eine verbesserte Vermehrung, wenn sie in Kontakt gebracht werden mit allogenen Lymphozyten aus peripherem Blut. Dieser Mechanismus könnte über Wechselwirkungen der CD2- und CD58-Moleküle gesteuert werden und die HIV-Produktion erhöhen. Die mit Herpesvirus saimiri transformierten Zellinien konnten stabil in Kultur für wenigstens ein Jahr gehalten werden und eine Ausscheidung von infektiösem Herpesvirus saimiri konnte nicht nachgewiesen werden. Es wird daher davon ausgegangen, daß das HIV-Virus nicht die Produktion von Herpesvirus saimiri induziert. Das erfindungsgemäße Verfahren ist besonders nützlich für die Vermehrung von HIV-Viren im großen Umfang, insbesondere von HIV-2-Isolaten aus asymptomatischen Patienten.

Die Zellinie Jurkat (Synonym JM) wurde 1976 etabliert, und zwar von einem Patienten dieses Namens. Sie wurde aus dem Blut eines 14-jährigen Jungen mit aktuter T-Lymphozyten-Leukämie (ALL) gezüchtet. Diese T-Zellinie wird in Suspension kultiviert, produziert nach Stimulation Interleukin-2 und trägt typische Oberflächenmarker von T-Zellen (CD2, CD3, CD4, CD5, CD6, CD7, CD34, CD45, TCR β). Jurkat wird als einfach kultivierbares Modell für menschliche T-Zellen in zahlreichen zellbiologischen und biochemischen Arbeiten eingesetzt. Die Zellinie ist u.a. bei Pawelec et al. (Eur. J. Immunol. 12:387-392, 1992) beschrieben.

### Beispiel 1

Es wurden verschiedene Zellinien, die das Molekül CD4 exprimieren, untersucht. Als Kontrolle wurde die Zellinie P1084 verwendet, die zwar das Oberflächenmarkermolekül CD8, nicht aber das Molekül CD4 exprimiert. Jede Kultur von 4 x 10⁶ Zellen wurde infiziert mit 1 ml zellfreier Virussuspension von HIV-1_{IIIB} (Popovic et al., Science, 224, S. 497-500, 1984), HIV-2_{ROD} (Clavel et al., Nature, 324, S. 691-695, 1986) oder HIV-2_{NEP} (Nick, S., Häfner, Y., Diniz, C.A., Jahn, G., Detection of HIV-2 infection in a Nepalese individual, AIFO im Druck, 1993), wie näher beschrieben in Böhm et al., Cytometry, 13, S. 259-266, 1992 und in Zellkulturplatten ausgesät.

Die Aktivitäten von Reverser Transkriptase (RT) der Suspensionen, die für die Infektion verwendet wurden, betrugen 20.000 cpm/ml für HIV-1_{IIIB}, 410.000 cpm/ml für HIV-2_{ROD} und 824.000 cpm/ml für HIV-2_{NEP}. Um die HIV-Replikation in diesen Kulturen zu bestimmen, wurde die Aktivität der Reversen Transkriptase in den Kulturüberständen gemessen (Böhm et al., Cytometry, 13, S. 259-266, 1992).

Der Virusstamm HIV-2_{NEP} wurde kürzlich von einem asymptomatischen nepalesischen Patienten isoliert. Dieses Isolat vermehrt sich nur auf MOLT-4 Klon 8 Zellen und auf Nabelschnurlymphozyten. Bisher waren alle Versuche fehlgeschlagen, andere T-Zellen oder Makrophagen-Linien zu infizieren, sogar wenn eine hohe infektiöse Dosis angewandt wurde.

Weiterhin wurde untersucht, ob mit Herpesvirus saimiri transformierte Zellen primäre Lymphozyten bei der Virusisolierung substituieren können. Die Patientenlymphozyten wurden abgetrennt und direkt kokultiviert mit CB-15 oder Kesting-Zellen. Alternativ hierzu wurden die Patientenzellen mit OKT-3 (Ortho Diagnostic Systems GmbH, Neckargemünd) drei Tage vorstimuliert (Eberlein et al., Virus Res., **19**, S. 153-162, 1991). Parallel hierzu wurde HIV-1 isoliert durch eine Standard-Lymphozyten-Kokultivierungstechnik (Eberlein et al., a.a.O.). Die HIV-1-Virusproduktion wurde anhand des Nachweises von p24 Antigen gemessen, und zwar in den Kulturüberständen an den Tagen 14, 21 und 28.

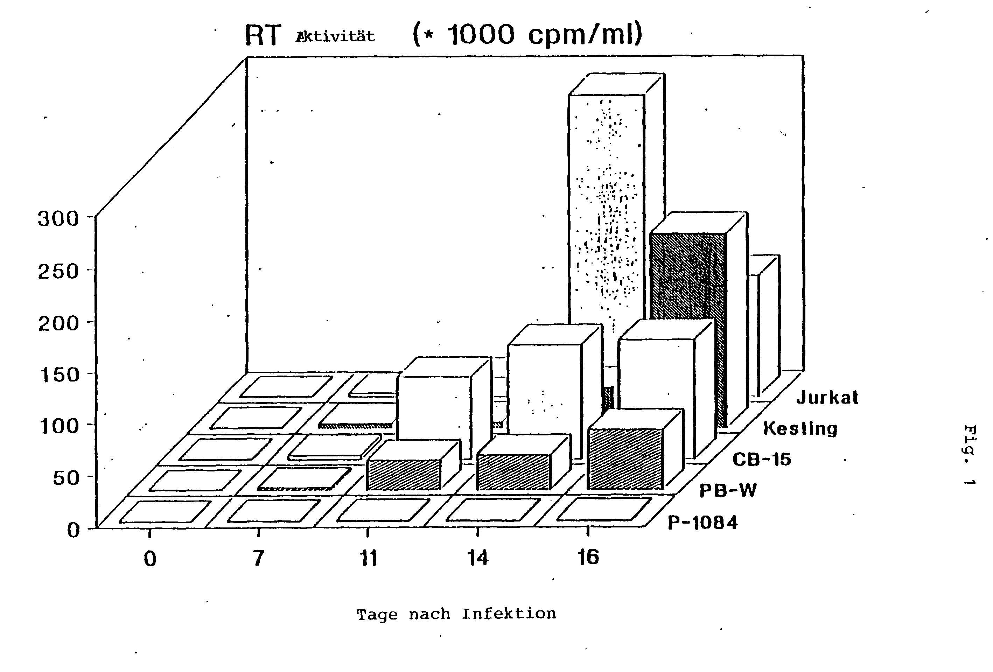

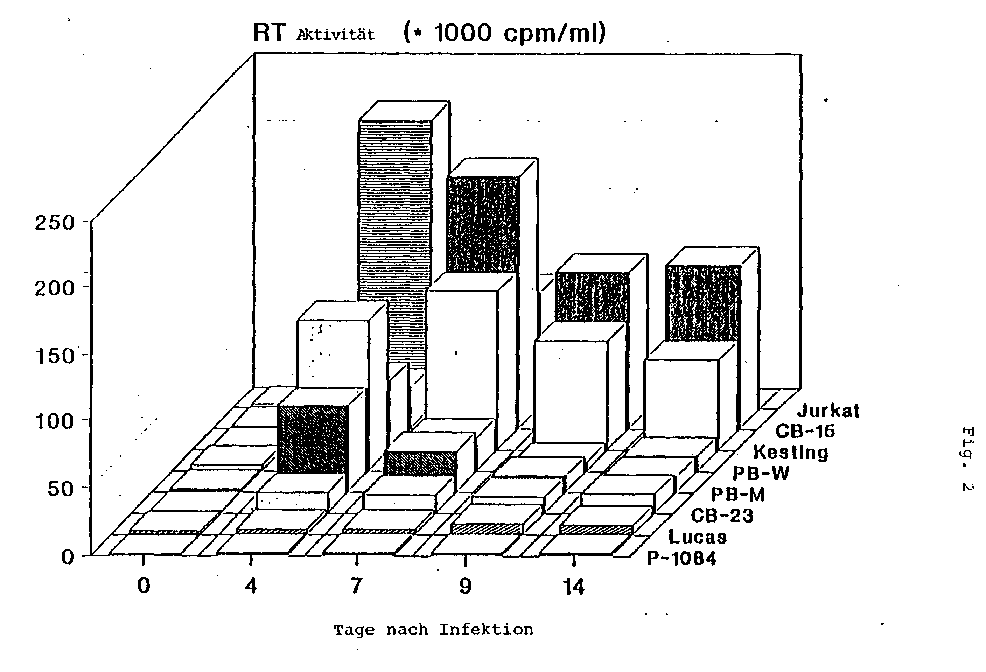

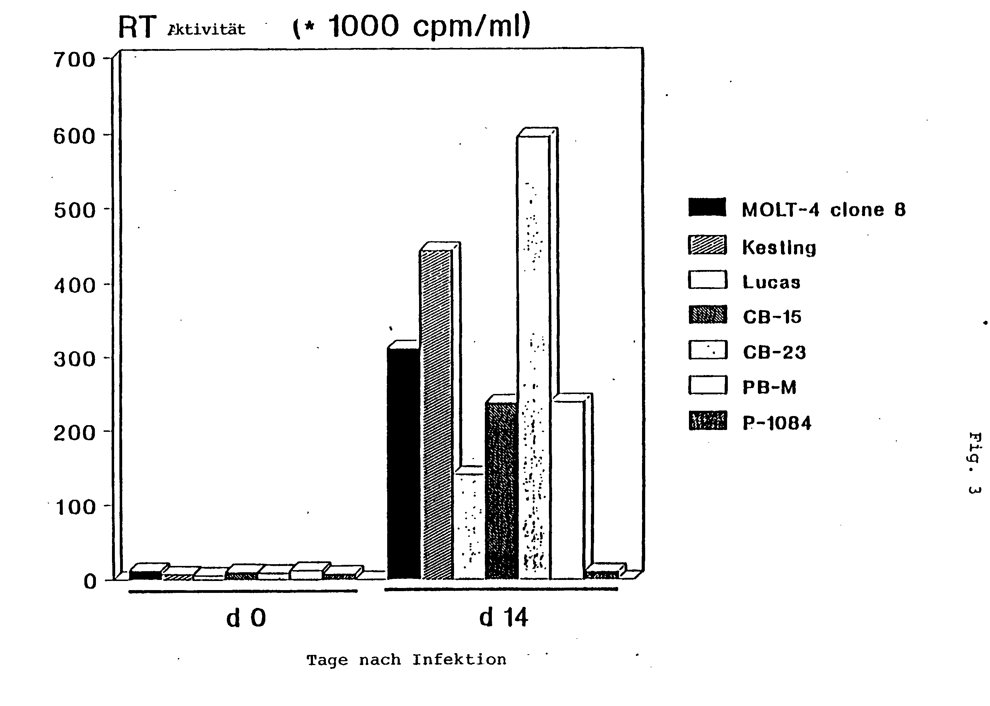

Die Typstämme HIV-1_{IIIB} und HIV-2_{ROD} vermehrten sich auf allen mit Herpesvirus saimiri transformierten Zellinien, die den Oberflächenmarker CD4 tragen und verursachten den Zelltod innerhalb von 16 Tagen. Die Ergebnisse der Untersuchungen sind in den Figuren 1 und 2 dargestellt. Die Kontrollzellinie P-1084, die den Oberflächenmarker CD4 nicht aufweist, war nicht anfällig gegenüber einer Infektion von HIV-1 oder HIV-2_{ROD} und überlebte. Die Zellinien Kesting und CB-15 produzierten die höchsten Mengen an HIV-1_{IIIB} und HIV-2_{ROD}. Obwohl die mit Herpesvirus saimiri transformierten Zellinien, die mit HIV-2_{NEP} infiziert wurden, keine zytopathischen Veränderungen aufwiesen, konnten hohe Aktivitäten an Reverser Transkriptase in den Überständen aller Zellinien aufgefunden werden, die den Zellmarker CD4 tragen. Hierbei handelt es sich um die Zellinien Kesting, Lucas, CB-15, CB-23 und PB-M. Die Ergebnisse sind in der Figur 3 dargestellt. Die Aktivität der Reversen Transkriptase, die aus CB-23-Zellen freigesetzt wurde, war sogar noch höher als die, die aus der Zellinie MOLT-4 Klon 8 freigesetzt wurde. Die Ergebnisse zeigen, daß mit Herpesvirus saimiri transformierte Zellinien, die den Zellmarker CD4⁺ aufweisen, ein sehr produktives System für anderenfalls nur schwach wachsende Viren vom HIV-2-Typ darstellen.

### Beispiel 2

Weiterhin wurde überprüft, ob CB-15 und/oder Kesting-Zellinien aus Nabelschnurblut gewonnene Lymphozyten für die anfängliche Isolierung von HIV-1-Viren ersetzen können. Lymphozyten aus dem peripheren Blut von vier Spendern mit Immunschwäche-Symptomen wurden kokultiviert mit Lymphozyten aus Nabelschnurblut, die stimuliert waren mit OKT-3 oder mit CB-15 oder Kesting-Zellinien. Die Ergebnisse sind in der nachfolgenden Tabelle 1 aufgeführt. In zwei Fällen (Spender H.F.R. und A.T.) war die HIV-1-Isolierung nur in Standard-Lymphozytenkokulturen (L/L) erfolgreich. Bei den Kulturen von dem Patienten B.K.H. dagegen konnten hohe Mengen an p24 in CB-15-Kulturen (L/CB-15) und Kesting-Kulturen (L/Kesting) nach 2 Wochen gemessen werden, während die Standard-Kokultivierung HIV-1 erst 2 Wochen später einen positiven Befund ergat Darüber hinaus war die Menge des produzierten viralen Antigens zehnmal höher in den Zellinien CB-15 und Kesting.

HIV-Virus wurde zweimal isoliert von dem Patienten D.P. im Abstand von sechs Wochen. Zuerst wurden die Spenderlymphozyten aus peripherem Blut direkt kokultiviert mit CB-15 und Kesting-Zellen. Bei dem zweiten Versuch wurden die Lymphozyten aus peripherem Blut vorstimuliert mit OKT-3 und dann kokultiviert (O/L/CB-15; O/L/Kesting). Das herkömmliche Kultursystem und die CB-15-Zellen waren in ähnlichem Maß erfolgreich. Die Isolierung auf Kesting-Zellen war nur erfolgreich, wenn vorstimulierte Lymphozyten aus peripherem Blut verwendet wurden. Zusammenfassend kann also festgestellt werden, daß, obwohl das HIV-1-Isolierungsergebnis heterogen war, in zwei Fällen die Verwendung von mit Herpesvirus saimiri transformierten Zellen dem Standardverfahren überlegen war.

**Tabelle 1**

| Replikation von frischen klinischen HIV-1-Isolaten mit Herpesvirus saimiri transformierten Zellen | | | | |
|---|---|---|---|---|
| Patient | Kultur | p24 [pg/ml] Tag 14 | p24 [pg/ml] Tag 21 | p24 [pg/ml] Tag 28 |
| H.F.R. | L/L | 63 | 39 | 3644 |
| | L/CB-15 | 13 | 0 | 0 |
| | L/Kesting | 0 | 0 | 0 |
| B.H.K. | L/L | 0 | 11 | 5328 |
| | L/CB-15 | 1487 | 15480 | 14208 |
| | L/Kesting | 1610 | 14430 | 11544 |
| D.P. | L/L | 336 | 1120 | 1127 |
| | L/CB-15 | 2498 | 1909 | 1510 |
| | L/Kesting | 433 | 145 | 17 |
| D.P. | L/L | 1277 | 1421 | 1376 |
| | O/L/CB-15 | 1199 | 1265 | 1554 |
| | O/L/Kesting | 155 | 1265 | 1554 |
| A.T. | L/L | 133 | 114 | 97 |
| | O/L/CB-15 | 12 | 0 | 0 |
| | O/L/Kesting | 53 | 0 | 0 |
| L/L: Standard Lymphozyten Kokultivierungstechnik (Eberlein et al., Virus Res. 19, S. 153-162, 1991) | | | | |
| L/CB-15; L/Kesting: Direkte Kokultivierung von Patienten Lymphozyten aus peripherem Blut und CB-15- oder Kesting-Zellen | | | | |
| O/L/CB-15; O/L/Kesting: Kokultivierung nach 3-Tage-Vorstimulierung mit OKT-3 | | | | |

Die in den Versuchen erhaltenen Ergebnisse werden in den anliegenden Figuren dargestellt.

Figur 1 zeigt die Replikation von HIV-1_{IIIB} in Herpesvirus saimiri transformierten T-Zellinien. 4x10⁶ Zellen wurden mit 1 ml HIV-1_{IIIB} infiziert, die eine Suspension von 20.000 cpm/ml an Reverser Transkriptase-Aktivitätenthielten, W1 beschrieben in Böhm et al., Cytometry, 13, S. 259-266, 1992. Zu den angegebenen Zeitpunkten wurden Proben des Kulturüberstandes auf Reverse Transkriptase-Aktivität überprüft.

Figur 2 zeigt die Replikation von HIV-2_{ROD} in den mit Herpesvirus saimiri transformierten T-Zellinien. Die Reverse Transkriptase-Aktivität der HIV-2_{ROD}-Suspension, die für die Infektion verwendet wurde, betrug 410.000 cpm/ml.

Figur 3 zeigt die Replikation von HIV-2_{NEP} in mit Herpesvirus saimiri transformierten T-Zellinien. Die Reverse Transkriptase-Aktivität der für die Infektion verwendeten HIV-2_{NEP}-Suspension betrug 824.000 cpm/ml.

## Patentansprüche

1. Verfahren zur Vermehrung von Immunschwäche verursachenden Viren vom HIV-Typ, **dadurch gekennzeichnet, daß** die Viren mit Hilfe von mit Herpesviren saimiri transformierten Lymphozyten vermehrt werden.

2. Verfahren nach Anspruch 1, **dadurch gekennzeichnet, daß** die menschlichen Lymphozyten mit Herpesvirus saimiri, mit Herpesviren der Gruppe C oder der Gruppe Non A- Non B transformiert werden.

3. Verfahren nach einem der vorhergehenden Ansprüche, **dadurch gekennzeichnet, daß** die Lymphozyten CD-4 positiv sind.

4. Verfahren nach einem der vorhergehenden Ansprüche, **dadurch gekennzeichnet, daß** die humanen Lymphozyten von Thymus, peripherem Blut, Nabelschnurblut, Lymphknoten, Lymphgefäßen, Milz oder Knochenmark stammen.

5. Verfahren nach einem der Ansprüche 1 bis 4, **dadurch gekennzeichnet, daß** es sich bei den Immunschwäche verursachenden Viren vom HIV-Typ um solche handelt, die von asymptomatischen, seropositiven Patienten stammen.

6. Verfahren nach einem der vorhergehenden Ansprüche, **dadurch gekennzeichnet, daß** mit Herpesvirus saimiri transformierte Lymphozyten mit anderen Zellen kokultiviert werden.

7. Verfahren der mit Herpesvirus saimiri transformierten Lymphozyten, die in einem Verfahren gemäß einem der Ansprüche 1 bis 6 eingesetzt werden, zur Vermehrung von Immunschwäche verursachenden Viren vom HIV-Typ.

## Claims

1. A process for replicating viruses of the HIV type which cause immune deficiency, wherein the viruses are replicated with the aid of lymphocytes which have been transformed with herpesvirus saimiri.

2. The process as claimed in claim 1, wherein the human lymphocytes are transformed with herpesvirus saimiri, with herpesviruses of Group C or of Group Non A - Non B.

3. The process as claimed in one of the preceding claims, wherein the lymphocytes are CD-4-positive.

4. The process as claimed in one of the preceding claims, wherein the human lymphocytes originate from thymus, peripheral blood, umbilical cord blood, lymph nodes, lymph vessels, spleen or bone marrow.

5. The process as claimed in one of claims 1 to 4, wherein the viruses of the HIV type which cause immune deficiency are those which originate from asymptomatic, seropositive patients.

6. The process as claimed in one of the preceding claims, wherein lymphocytes transformed with herpesvirus saimiri are co-cultivated with other cells.

7. The use of the lymphocytes transformed with herpesvirus saimiri, which lymphocytes are employed in a process as claimed in one of claims 1 to 6, for replicating viruses of the HIV type which cause immune deficiency.

## Revendications

1. Procédé pour la multiplication de virus du type VIH provoquant l'immunodéficience, **caractérisé en ce qu'**on multiplie les virus à l'aide de lymphocytes transformés par des *Herpesvirus.*

2. Procédé selon la revendication 1, **caractérisé en ce qu'**on transforme les lymphocytes humains par l'*Herpesvirus saimiri,* par des *Herpesvirus* du groupe C ou du groupe Non A-Non B

3. Procédé selon l'une des revendications précédentes, **caractérisé en ce que** les lymphocytes sont CD-4 positifs.

4. Procédé selon l'une des revendications précédentes, **caractérisé en ce que** les lymphocytes humains proviennent du thymus, du sang périphérique, du sang du cordon ombilical, des ganglions lymphatiques, des vaisseaux lymphatiques, de la rate ou de la moëlle osseuse.

5. Procédé selon l'une des revendications 1 à 4, **caractérisé en ce qu'**il s'agit, pour les virus du type VIH provoquant l'immunodéficience, de virus qui proviennent de patients séropositifs, asymptotiques.

6. Procédé selon l'une les revendications précédentes, **caractérisé en ce qu'**on cocultive les lymphocytes transformés par l'*Herpesvirus saimiri* avec d'autres cellules.

7. Procédé utilisant des lymphocytes transformés par l'*Herpesvirus saimiri*, utilisés dans un procédé selon l'une des revendications 1 à 6, pour la multiplication de virus de type VIH provoquant l'immunodéficience.
